# EUROPEAN PATENT SPECIFICATION

(11) **EP 0 462 701 B1**
(45) Date of publication and mention of the grant of the patent: **14.12.1994**
(21) Application number: 91304512.6
(22) Date of filing: 20.05.1991
(51) Int. Cl.: A61M 16/06

(54) **Nasal mask**
Nasenmaske
Masque nasal

(30) Priority: 21.05.1990 AU 228/90
(43) Date of publication of application: 27.12.1991
(73) Proprietor: THE UNIVERSITY OF SYDNEY, Sydney, New South Wales 2006 (AU)
(72) Inventor: Sullivan, Colin Edward, Birchgrove, New South Wales 2041 (AU); Bruderer, Jakob Walter, Kingsford, New South Wales 2032 (AU)
(74) Representative: Ben-Nathan, Laurence Albert

(56) References cited:
- DE-A- 3 707 952
- DE-B- 1 104 122
- GB-A- 697 762
- GB-A- 848 215
- US-A- 1 635 275

## Description

This invention relates to a nasal mask and, in particular, to a mask that has the facility to conform to the facial contours of a wearer.

Nasal masks currently are employed for various purposes, including for the delivery of oxygen to persons who suffer lung disease or who are exposed to rarefied atmospheres, for administering anaesthesic gases and for delivering pressurised air to persons who suffer from such disorders as sleep apnea. The masks usually are moulded from a relatively soft, resilient plastics material and they are shaped during manufacture to match the facial contours of an average intended wearer. However, a problem with the known types of masks is that, because individuals vary so much from the average, the masks must be forced against their inherent resiliency to deform and so adapt to the shapes of the users in order to avoid gas leakage. This requires that the masks be secured firmly by retaining straps or harnesses in order to prevent air leakage and, depending on the degree of deformation required in any given case, may produce discomfort, irritation or even ulceration of the upper lip and/or the nasal bridge where there is little cushioning from subcutaneous tissue. Thus, the retaining force normally is distributed over a relatively small sealing area defined by the peripheral edge of the mask and this causes a relatively high localised pressure to be exerted on the face of the wearer. Also, because the sealing area is relatively small in the currently employed masks, minor dislocation of a mask may produce a significant leakage path for gas.

Attempts have been made to overcome the above difficulties by shaping perimeter edges of some masks in such a manner that the edges tend to move with a rolling action when a mask is pushed into contact with a wearer's face. This shaping permits differential movement around the perimeter of a mask and facilitates adaptation of the mask to the facial contours of the wearer.

Also, United States patent number 4,971,051 discloses a mask which has been developed in an attempt to provide both comfortable and conformable perimeter sealing, the mask having a flexible pneumatic cushion formed around its perimeter and/or being provided with a so-called flap ring which is formed as an adjunct to the mask. The flap ring comprises a flexible membrane which is affixed to the perimeter of the mask and it acts in the manner of a flap valve when blown against a wearer's face by air delivered to the interior of the mask.

A problem which is inherent in a mask of the type disclosed in U.S. patent number 4,971,051 is that the flap ring is constrained by its attachment to the perimeter seal of the mask and its freedom to adapt to facial contours is limited. Moreover, again because the flap ring is attached to the perimeter seal of the mask, any movement of the perimeter seal may cause movement of the flap ring relative to the wearer's face and thereby open a path through which gas may leak. Similarly, movement of the flap ring may cause stresses to be imposed on the perimeter seal, so that a path may be created through which gas may leak.

DE-B-1104122 discloses a prior art mask which is intended to completely cover the nose and the mouth regions of a wearer. The mask has a large opening which receives the nose and mouth of the wearer and the opening has a perimeter seal which is acted upon in the manner of a flap to close against the face of the wearer when the interior of the mask is pressurised.

In contrast with the prior art masks, the present invention provides a nasal mask according to claim 1 or claim 2.

The face contacting portion of the mask according to the present invention may be considered as having a shape which is unrelated to that of the intended wearer until such time as the mask is charged with gas and fitted to the wearer. However, when so charged and fitted, the membrane is caused to distend, to overlay the covered facial regions and to conform three-dimensionally with the facial contours of the wearer. In so doing, the membrane is caused to deform locally to accommodate individual facial projections and depressions, and distortions of the type that might otherwise create peripheral air leakage passages are thereby avoided. This is to be contrasted with the prior art masks which are moulded or are otherwise constructed with a clearly defined perimeter shape which is intended to match that of an intended wearer and which, except in the event of a perfect fit, must be distorted to match the features of the wearer.

Thus, the mask in accordance with the present invention may be viewed as a self-conforming mask which, by overlaying and conforming to the shape of the wearer provides an inherently large self-sealing area.

The gas supply port and the retaining means may be secured to or be formed integrally with the distendable membrane itself. However, the distendable membrane is preferably mounted to a shell which is shaped to overfit the nose region at least of the wearer and which co-operates with the membrane to define the chamber. When this preferred arrangement is employed the retaining means would normally be connected to the shell and the gas supply port would normally be provided in a wall of the shell.

The shell is preferably moulded from a rigid plastics material and the membrane, which normally would be moulded from an elastic plastics material, is preferably removably mounted to a peripheral portion of the shell.

The distendable membrane is preferably secured to or moulded as an extension of a connector moulding which is arranged to mount the membrane to the shell. The connector moulding may be configured to contact facial regions of the wearer and, thus, be employed to locate the mask in a desired position on the face of the wearer. However, it is intended that the connector moulding should not interfere in any way with the face conforming feature of the distendable membrane.

When the membrane is formed with a single orifice, such orifice may be formed as an aperture which is shaped and sized to permit unimpeded communication between the chamber and the nasal passages of a wearer to whom the mask is fitted. Alternatively, the aperture may be shaped and sized to permit communication between the chamber and the naresmouth region of the wearer, so that the mask may be employed as a full face mask. As a further alternative, two apertures may be provided for aligning with the respective nasal passages. However, when the membrane is formed with two orifices they preferably will be provided within respective nipples which are shaped and positioned to project into the nasal passages of the wearer to whom the mask is fitted. The nipples are preferably moulded integrally with the remaining portion of the membrane, so that they may be positioned and shaped elastically to match the wearer.

The invention will be more fully understood from the following description of two embodiments of a nasal mask which is suitable for use in administering air to persons who suffer from sleep apnea. The description is provided with reference to the accompanying drawings in which:
Figure 1 shows a schematic illustration of a continuous positive airway pressure (CPAP) circuit in which the mask may be used,

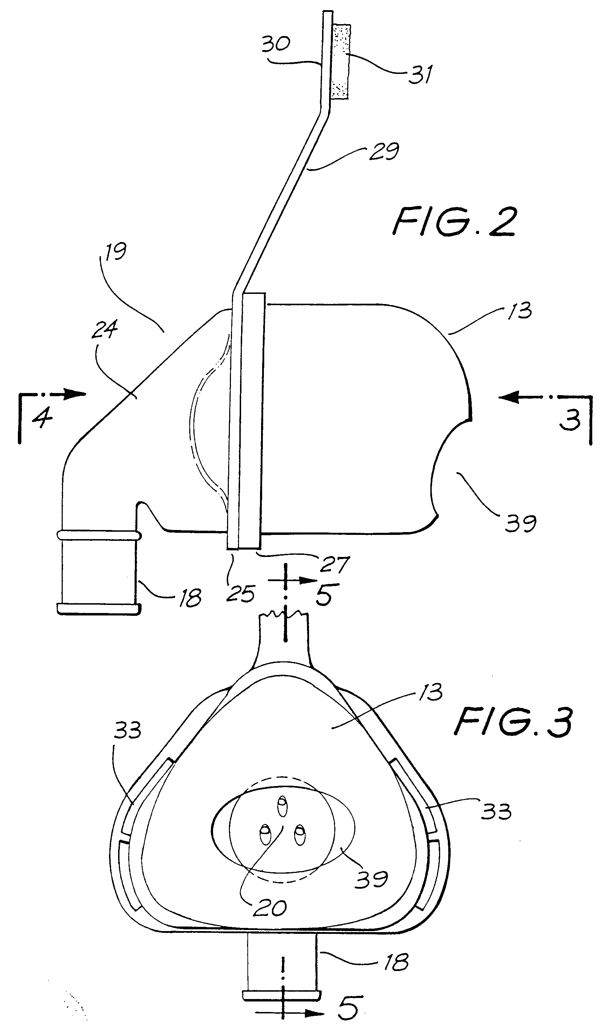

Figure 2 shows a side elevation view of one embodiment of the mask,
Figure 3 shows a front elevation view of the mask as illustrated in Figure 2 and as seen in the direction of arrow 3 shown in Figure 2,
Figure 4 shows a rear elevation view of the mask as illustrated in Figure 2 and as seen in the direction of arrow 4 shown in Figure 2,
Figure 5 shows a sectional elevation view of a portion of the mask as seen in the direction of section plane 5-5 shown in Figure 3,
Figure 6 shows a side elevation of a second embodiment of the mask,
Figure 7 shows a front elevation view of the mask as illustrated in Figure 6 and as seen in the direction of arrow 7 as shown in Figure 6, and
Figures 8A, B and C illustrate sequentially the fitting of the mask as illustrated in Figures 1-5 to a wearer.

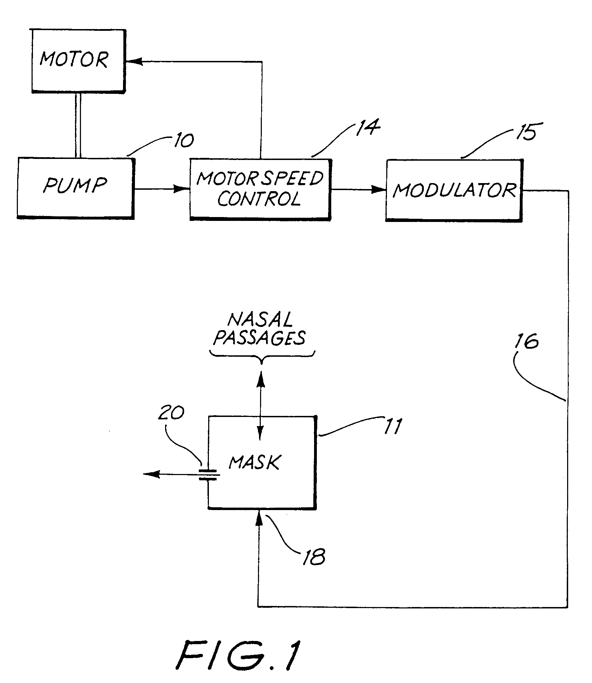

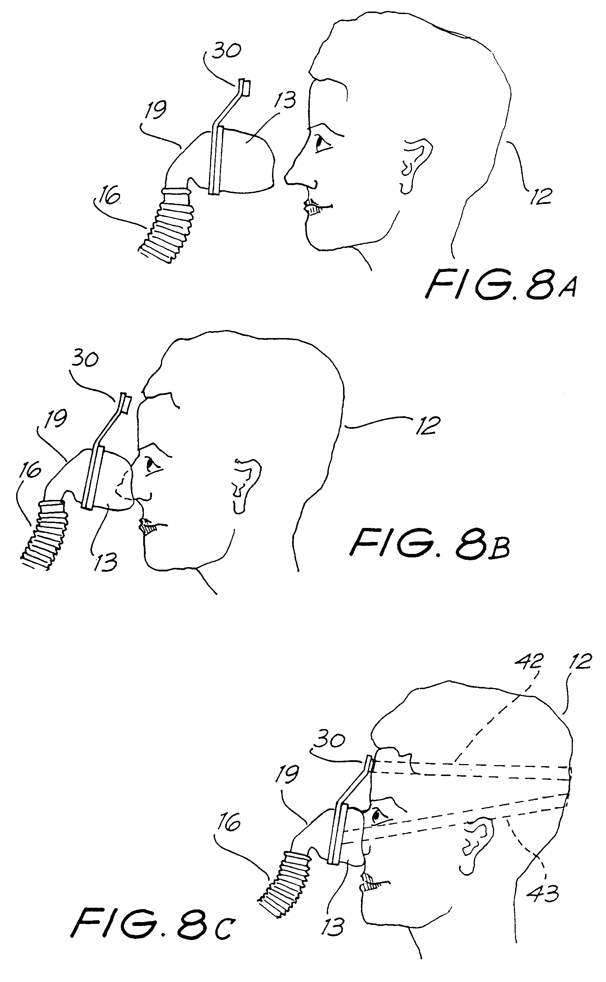

As illustrated in Figure 1, the CPAP circuit comprises a motor driven pump 10 which is arranged to provide an air supply to a mask 11 which is fitted to a wearer 12 in the manner shown in Figure 8. The air is supplied at a flow rate greater than the rate at which the air would normally be inspired in order to create backpressure within the circuit, whilst the wearer maintains regular breathing, in order that a membrane portion 13 of the mask will be caused to distend in the manner which is to be described in greater detail below. A motor speed controller 14 is located in circuit with the pump 10 and functions effectively to control the air pressure in the system to meet the requirements of individual persons. Also, a modulator 15 may optionally be located in circuit with the pump for modulating the pressure at which the air is delivered to the mask. The modulator is arranged to provide pressure modulation to a depth within the range of 5% to 50% and at a frequency in the range 5Hz to 60Hz in order to effect stimulation of respiratory tract muscles in the wearer 12.

The air is conveyed to the mask 11 by way of a delivery line 16 and a supply port 18 in a shell portion 19 of the mask.

As illustrated, the mask 11 is in general shaped to cover the nose area of the person 12 to whom the air is to be supplied and does not cover the mouth of the person. Thus, air from the supply port 18 is directed into the upper air passage of the person by way of the mask 11 and the person's nasal passages. However, it will be understood that the primary feature of the mask, namely the distendable membrane 13 may be incorporated in a full face mask, in which case the mask will be shaped to cover both the nose and mouth of the person.

Excess air, that is air which is not inspired during normal breathing, is vented to the atmosphere by way of a multi-aperture orifice 20 in the shell portion 19 of the mask 11. The orifice 20 presents a resistance to the air flow, so that a continuous positive pressure is maintained within the circuit and, thus, within the mask. By maintaining this pressure, the air which is breathed by the person 12 to whom the mask is fitted causes a pressure to be maintained in the upper airway of the person during normal inspiration and expiration.

Air which is expired by the person 12 is also vented to atmosphere by way of the orifice 20.

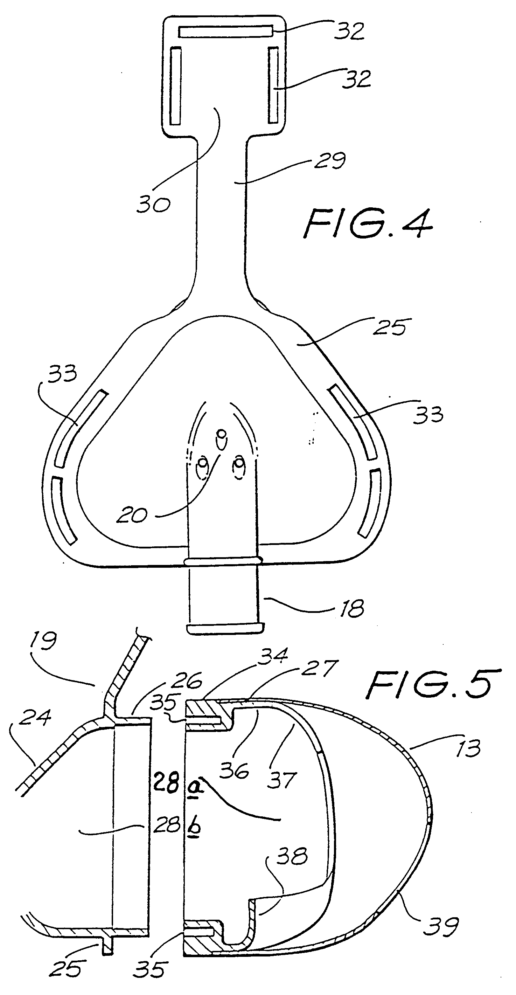

The shell 19 of the mask is moulded from a rigid plastics material and it has a body portion 24 around which a flange 25 extends. As can best be seen from Figure 5, a lip 26 projects forwardly of the body portion 24 and a connector moulding 27, which carries the distendable membrane portion 13, overfits the lip 26. The interior of the shell 19 forms a cavity 28b which is defined in part by the membrane 13 and the connector moulding 27, and air is admitted to the cavity 28b from the support port 18.

An arm 29 extends upwardly from the body portion 24 of the shell and is angled such that an upper head engaging portion 30 of the arm will contact the forehead of the person 12 to whom the mask is fitted. A pad 31 of cushioning material is adhered to the head engaging portion 30 and slots 32 are located in that portion to accommodate harness straps 42 which are used to secure the mask to the head of the person 12. Additional slots 33 are formed within the peripheral flange 25 of the shell body portion 24 for receiving further harness straps 43.

The connector moulding 27 has a peripheral flange 34 in which a circumferential recess 35 is located. The recess 35 is positioned to overfit the shell lip 26 and the recess serves to hold the moulding 27 captive to the shell 19. The moulding 27 might be configured to provide an elementary support for the membrane 13 or, in an alternative arrangement, the membrane might be formed integrally with the moulding 27. However, as illustrated, the moulding 27 is formed separately from the membrane 13 and includes an integral wall 36 which is profiled at region 37 to locate around the nasal bridge of the wearer 12 and at region 38 to fit against the upper lip of the wearer.

The membrane 13 is formed as a thin-walled balloon-like pocket, and is moulded from a soft, flexible plastics material. The membrane is adhered to the wall 36 of the moulding 27, and an aperture 39 is formed within the membrane 13. The aperture 39 is shaped, positioned and sized to provide air passage communication between the chamber 28a of the mask and the nasal passages of the wearer 12, and various size membranes 13 may be provided to accommodate different size wearers, for example adults and children.

A small orifice (not shown in the drawings, may be provided in the membrane to act as a vent for any moisture that might otherwise build up between the mask and the wearer's face.

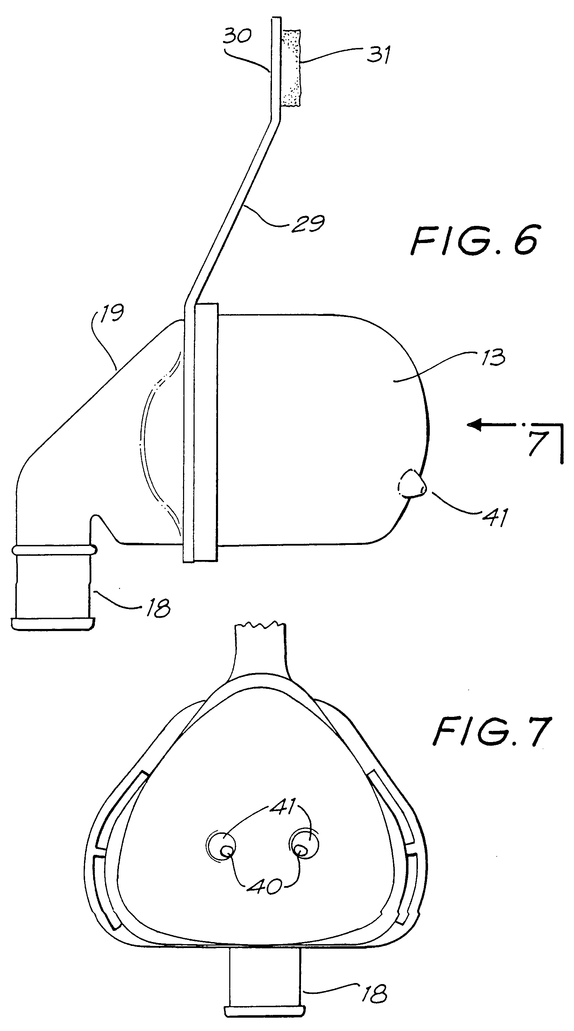

In the alternative arrangement shown in Figures 6 and 7, the air passage between the chamber 18 of the mask and the nasal passages of the wearer is provided by way of two orifices 40 which are located within respective nipples 41. The nipples themselves are shaped, positioned and sized to fit within the nasal passages of the wearer 12.

As shown in the sequential illustrations of Figures 8A to 8C, when fitting the nasal mask to the wearer 12, the mask is first connected to the pressurised air supply so that the membrane 13 is caused to billow or distend outwardly from the shell 19. A face contacting portion of the mask is then placed in contact with the wearer's nose and is positioned such that the aperture 39 or the nipples 41 align with the nasal passages. In this condition, as shown in Figure 8B of the drawings, the membrane 13 will begin moulding around the nose of the wearer.

As the mask is pushed further into position, the shell 19 is moved closer to the wearer's nose and, under the influence of the air pressure within the chamber 28a, the membrane 13 moulds completely around the nose of the wearer and conforms to the facial contours which are actually overlayed by the membrane. The mask is then pushed into its final position, as indicated in Figure 8C and the retaining harness, indicated by the chain dotted lines 42 and 43, is positioned around the wearer's head to retain the mask in position.

## Claims

1. A nasal mask which comprises a face contacting portion in the form of an elastomeric material membrane (13) which is capable of distending and which is shaped when distended to define a balloon-like chamber (28a), the chamber (28a) having a thin walled externally convex end region which is arranged in use of the mask to balloon outwardly and to be depressed by and accommodate the nose of a wearer, a gas supply port (18) communicating with the chamber (28a) and connectable to a supply of pressurized gas which, when admitted to the chamber, causes the end region (13) to balloon outwardly, retaining means (42,43) for holding the face contacting portion in contact with the face of the wearer whereby the end region of the chamber is caused to overlay at least a nose region of the wearer's face and, under the influence of the pressurised gas, to conform and seal three-dimensionally with the contours of the overlayed region, and at least one orifice (39) formed within the membrane, the orifice being shaped and positioned to admit gas from the chamber (28a) to the nasal passages of the wearer.

2. A nasal mask which comprises a shell (19) which is shaped to overfit at least a nose region of the face of a wearer and which defines a cavity (28b), a face contacting portion in the form of an elastomeric material membrane (13) which is mounted to the shell, the membrane being capable of distending and being shaped when distended to form a balloon-like chamber (28a) in fluid communication with the cavity (28b) in the shell, the chamber (28a) having a thin walled externally convex end region which is arranged in use of the mask to balloon outwardly and to be depressed by and to accommodate the nose of the wearer, a gas supply port (18) in the shell (19) and communicating with the chamber (28a) by way of the cavity (28b) in the shell, the port (18) being connectable to a supply of pressurised gas which, when admitted to the chamber, causes the end region of the chamber to balloon outwardly from the shell (19), retaining means (42,43) for holding the face contacting portion in contact with the face of the wearer whereby the end region of the chamber is caused to overlay at least the nose region and, under the influence of the pressurised gas, to conform and seal three-dimensionally with the contours of the overlayed region, and at least one orifice (39) formed within the membrane (13), the orifice being shaped and positioned to admit gas from the chamber (28a) to the nasal passages of the wearer.

3. A nasal mask as claimed in claim 2 wherein the shell (19) is moulded from a rigid plastics material.

4. A nasal mask as claimed in claim 2 or claim 3 wherein the membrane (13) is removably mounted to the shell (19).

5. A nasal mask as claimed in claim 1 wherein the membrane (13) is removably mounted to a shell (19), wherein the shell (19) defines a cavity (28b) in fluid communication with the chamber (28a) formed by the membrane and wherein the gas supply port (18) is formed as a part of the shell.

6. A nasal mask as claimed in claim 4 or claim 5 wherein the membrane is mounted to the shell by way of a connector moulding (27).

7. A nasal mask as claimed in claim 6 wherein the membrane (13) is formed integrally with the connector moulding (27).

8. A nasal mask as claimed in any one of claims 2 to 7 wherein the end region of the chamber (28a) extends outwardly from a relatively thick wall region (36), the wall region being configured to conform with the facial regions of the wearer.

9. A nasal mask as claimed in claim 6 wherein the membrane (13) is formed separately from and is adhered to the connector moulding (27).

10. A nasal mask as claimed in claim 7 or claim 9 wherein the connector moulding (27) is located predominantly within the membrane (13) and is configured to conform with facial features of the wearer.

11. A nasal mask as claimed in any one of the preceding claims wherein only one said orifice (39) is formed within the membrane (13), the orifice being in the form of an aperture which is shaped and sized to permit communication between the chamber (28a) and both nasal passages of the wearer.

12. A nasal mask as claimed in any one of claims 1 to 10 wherein only one said orifice (39) is formed within the membrane (13), the orifice being in the form of an aperture which is shaped and sized to provide communication between the chamber (28a) and both the nasal passages and the mouth of the wearer.

13. A nasal mask as claimed in any one of claims 1 to 10 wherein two said orifices (40) are formed within the membrane (13), the orifices being provided within respective nipples (41) which are shaped and positioned to project into the nasal passages of the wearer when the mask is fitted to the wearer.

14. A nasal mask as claimed in any one of the preceding claims connected in circuit with a continuous positive airway pressure (CPAP) system which comprises a motor driven pump (10) and means (14) for controlling the speed of the motor.

## Patentansprüche

1. Nasenmaske, die einen Abschnitt für den Kontakt mit dem Gesicht in Form einer Membran (13) aus einem elastomeren Material, die sich ausdehnen kann, und die, wenn sie gedehnt ist, so geformt ist, daß sie eine ballonähnliche Kammer (28a) definiert, wobei diese Kammer (28a) einen dünnwandigen äußeren konvexen Endabschnitt aufweist, der bei Verwendung der Maske so angeordnet ist, daß er durch die Nase des Trägers nach außen gewölbt und heruntergedrückt wird und die Nase aufnimmt, eine Gaszufuhröffnung (18), die mit der Kammer (28a) in Verbindung steht und mit der Zufuhr von komprimiertem Gas verbunden werden kann, das beim Eintritt in die Kammer bewirkt, daß sich der Endbereich (13) nach außen wölbt, Halteeinrichtungen (42, 43) zum Halt des Abschnittes für den Kontakt mit dem Gesicht in Kontakt mit dem Gesicht des Trägers, wodurch der Endbereich der Kammer zumindest auf dem Nasenbereich des Gesichts des Trägers liegt und unter dem Einfluß des komprimierten Gases mit den Konturen dieses überlagerten Bereiches übereinstimmt und diese dreidimensional abdichtet, und mindestens eine Öffnung (39) umfaßt, die in dieser Membran ausgebildet ist, wobei diese Öffnung so geformt und angeordnet ist, daß Gas aus der Kammer (28a) in die Nasenöffnungen des Trägers eintreten kann.

2. Nasenmaske, die eine Ummantelung (19), die so geformt ist, daß sie zumindest auf dem Nasenbereich des Gesichts des Trägers sitzt, und die einen Hohlraum (28b) definiert, einen Abschnitt für den Kontakt mit dem Gesicht in Form einer Membran (13) aus einem elastomeren Material, die an der Ummantelung angebracht ist, wobei sich die Membran ausdehnen kann und, wenn sie gedehnt ist, so geformt ist, daß in der Ummantelung eine ballonähnliche Kammer (28a) in Fluidverbindung mit dem Hohlraum (28b) geformt wird, wobei die Kammer (28a) einen dünnwandigen äußeren konvexen Endabschnitt aufweist, der bei Verwendung der Maske so angeordnet ist, daß er von der Nase des Trägers nach außen gewölbt und heruntergedrückt wird und diese aufnimmt, eine Gaszufuhröffnung (18) in der Ummantelung (19), die durch den Hohlraum (28b) in der Ummantelung mit der Kammer (28a) in Verbindung steht, wobei diese Öffnung (18) mit der Zufuhr des komprimierten Gases verbunden werden kann, das beim Eintritt in die Kammer bewirkt, daß sich der Endbereich der Kammer von der Ummantelung (19) nach außen wölbt, Halteeinrichtungen (42, 43) zum Halt des Abschnittes für den Kontakt mit dem Gesicht in Kontakt mit dem Gesicht des Trägers, wodurch der Endbereich der Kammer zumindest auf dem Nasenbereich liegt und unter dem Einfluß des komprimierten Gases mit den Konturen dieses überlagerten Bereiches übereinstimmt und diese dreidimensional abdichtet, und mindestens eine Öffnung (39) umfaßt, die in der Membran (13) ausgebildet ist, wobei diese Öffnung so geformt und angeordnet ist, daß das Gas aus der Kammer (28a) in die Nasenöffnungen des Trägers eintreten kann.

3. Nasenmaske nach Anspruch 2, worin die Ummantelung (19) aus einem starren Kunststoffmaterial geformt ist.

4. Nasenmaske nach Anspruch 2 oder 3, worin die Membran (13) abnehmbar an der Ummantelung (19) angebracht ist.

5. Nasenmaske nach Anspruch 1, worin die Membran (13) abnehmbar an der Ummantelung (19) angebracht ist, wobei diese Ummantelung (19) einen Hohlraum (28b) in Fluidverbindung mit der Kammer (28a) definiert, die durch die Membran gebildet wird, und worin die Gaszufuhröffnung (18) als Teil der Ummantelung ausgebildet ist.

6. Nasenmaske nach Anspruch 4 oder 5, worin die Membran durch ein Verbindungsformteil (27) an der Ummantelung angebracht ist.

7. Nasenmaske nach Anspruch 6, worin die Membran (13) einstückig mit dem Verbindungsformteil (27) ausgebildet ist.

8. Nasenmaske nach einem der Ansprüche 2 bis 7, worin sich der Endbereich der Kammer (28a) vom relativ dicken Wandbereich (36) nach außen erstreckt, wobei dieser Wandbereich so geformt ist, daß er mit den Gesichtsbereichen des Trägers übereinstimmt.

9. Nasenmaske nach Anspruch 6, worin die Membran (13) getrennt vom Verbindungsformteil (27) ausgebildet ist und an diesem haftet.

10. Nasenmaske nach Anspruch 7 oder 9, worin das Verbindungsformteil (27) vorwiegend in der Membran (13) angeordnet und so gestaltet ist, daß es mit den Gesichtsmerkmalen des Trägers übereinstimmt.

11. Nasenmaske nach einem der vorstehenden Ansprüche, wobei nur eine der Öffnungen (39) in der Membran (13) ausgebildet ist, wobei diese Öffnung in Form eines Lochs vorliegt, das so geformt und bemessen ist, daß es eine Verbindung zwischen der Kammer (28a) und beiden Nasenlöchern des Trägers erlaubt.

12. Nasenmaske nach einem der Ansprüche 1 bis 10, wobei nur eine der Öffnungen (39) in der Membran (13) ausgebildet ist, wobei diese Öffnung in Form eines Lochs vorliegt, das so geformt und bemessen ist, daß es eine Verbindung zwischen der Kammer (28a) und beiden Nasenlöchern und dem Mund des Trägers bietet.

13. Nasenmaske nach einem der Ansprüche 1 bis 10, wobei beide Öffnungen (40) in der Membran (13) ausgebildet sind, wobei diese Öffnungen in entsprechenden Nippeln (41) vorgesehen sind, die so geformt und angeordnet sind, daß sie in die Nasenlöcher des Trägers ragen, wenn die Maske dem Träger angepaßt wurde.

14. Nasenmaske nach einem der vorstehenden Ansprüche, die mit einem Schaltkreis eines Systems für einen Luftweg mit kontinuierlichem Überdruck (CPAP) verbunden ist, das eine von einem Motor betätigte Pumpe (10) und eine Einrichtung (14) zur Regelung der Geschwindigkeit dieses Motors umfaßt.

## Revendications

1. Un masque nasal qui comprend une partie de contact avec le visage sous la forme d'une membrane (13) en un matériau élastomère, qui est capable de se distendre et qui est conformée lorsqu'elle est distendue pour définir une chambre (28a) semblable à un ballon, la chambre (28a) ayant une zone terminale extérieurement convexe à paroi mince, qui est agencée lorsque le masque est en service pour se gonfler vers l'extérieur et pour être enfoncée par le nez d'un porteur et s'y adapter, un raccord d'alimentation en gaz (18) communiquant avec la chambre (28a) et étant susceptible d'être relié à une source de gaz sous pression, lequel, quand il est admis dans la chambre, oblige la région terminale (13) à se gonfler vers l'extérieur, des moyens de maintien (42, 43) pour retenir la partie de contact avec le visage et qui est en contact avec le visage du porteur, de telle manière que la zone terminale de la chambre soit obligée de recouvrir au moins une région nasale du visage du porteur, et, sous l'action du gaz sous pression, de s'adapter et de s'appliquer de manière étanche et tridimensionnelle aux contours de la zone recouverte, ainsi qu'au moins un orifice (39) ménagé dans la membrane, cet orifice étant conformé et situé pour amener le gaz provenant de la chambre (28a) aux conduits nasaux du porteur.

2. Un masque nasal qui comprend une coquille (19) qui est conformée pour recouvrir en s'y adaptant au moins une région nasale du visage d'un porteur et qui définit: une cavité (28b); une partie de contact avec le visage ayant la forme d'une membrane (13) en un matériau élastomère et qui est montée sur la coquille, cette membrane étant capable de se distendre et étant conformée lorsqu'elle est distendue pour définir une chambre (28a) semblable à un ballon et en communication de fluide avec la cavité (28b) dans la coquille, la chambre (28a) ayant une région terminale extérieurement convexe à paroi mince, qui est agencée lorsque le masque est en service pour se gonfler vers l'extérieur et pour être enfoncée par le nez du porteur et s'y adapter; un raccord d'alimentation en gaz (18) dans la coquille (19) et communiquant avec la chambre (28a) par l'intermédiaire de la cavité (28b) dans la coquille, le raccord (18) étant susceptible d'être connecté à une source de gaz sous pression, lequel, quand il est admis dans la chambre, oblige la zone terminale de la chambre à se gonfler vers l'extérieur à partir de la coquille (19); des moyens de maintien (42, 43) pour retenir la partie de contact avec le visage et qui est en contact avec le visage du porteur, de telle manière que la zone terminale de la chambre soit obligée de recouvrir au moins la région nasale, et, sous l'action du gaz sous pression, de s'adapter et de s'appliquer de manière étanche et tridimensionnelle aux contours de la zone recouverte, ainsi qu'au moins un orifice (39) ménagé dans la membrane, cet orifice étant conformé et situé pour amener le gaz provenant de la chambre (28a) aux conduits nasaux du porteur.

3. Un masque nasal selon la revendication 1, dans lequel la coquille (19) est moulée à partir d'une matière plastique rigide.

4. Un masque nasal selon la revendication 2 ou la revendication 3, dans lequel la membrane (13) est fixée de manière amovible à la coquille (19).

5. Un masque nasal selon la revendication 1, dans lequel la membrane (13) est fixée de manière amovible à une coquille (19), dans lequel la coquille (19) définit une cavité (28b) en communication de fluide avec la chambre (28a) formée par la membrane et dans lequel le raccord (18) d'alimentation en gaz est réalisé comme une partie de la coquille.

6. Un masque nasal selon la revendication 4 ou la revendication 5, dans lequel la membrane est fixée à la coquille au moyen d'une moulure de connexion (27).

7. Un masque nasal selon la revendication 6, dans lequel la membrane (13) est formée monobloc avec la moulure de connexion (27).

8. Un masque nasal selon l'une des revendications 2 à 7, dans lequel la région terminale de la chambre (28a) s'étend vers l'extérieur à partir d'une zone (36) à paroi relativement épaisse, cette zone de paroi étant conformée pour épouser les régions faciales du porteur.

9. Un masque nasal selon la revendication 6, dans lequel la membrane (13) est réalisée séparément de la moulure de connexion (27) et est fixée ou adhérisée à celle-ci.

10. Un masque nasal selon la revendication 7 ou la revendication 9, dans lequel la moulure de connexion (27) est disposée principalement dans la membrane (13) et est conformée pour épouser les particularités faciales du porteur.

11. Un masque nasal selon l'une quelconque des revendications précédentes, dans lequel seul ledit orifice (39) est formé dans la membrane (13), l'orifice ayant la forme d'une ouverture dont la forme et les dimensions permettent à la chambre (28a) et aux conduits nasaux du porteur de communiquer.

12. Un masque nasal selon l'une quelconque des revendications 1 à 10, dans lequel seul ledit orifice (39) est formé dans la membrane (13), l'orifice ayant la forme d'une ouverture dont la forme et les dimensions permettent à la chambre (28a) et, à la fois, aux conduits nasaux et à la bouche du porteur, de communiquer.

13. Un masque nasal selon l'une quelconque des revendications 1 à 10, dans lequel deux orifices (40) sont formés dans la membrane (13), ces orifices étant munis d'embouts (41) correspondants qui sont conformés et positionnés pour faire saillie dans les conduits nasaux du porteur lorsque le masque est monté sur le porteur.

14. Un masque nasal selon l'une quelconque des revendications précédentes, relié en série à un système continu de pression de ventilation positive (CPAP) qui comporte une pompe (10) entraînée par un moteur et des moyens (14) de commande de la vitesse de ce moteur.
